# EUROPEAN PATENT APPLICATION

(11) **EP 4 191 598 A1**
(43) Date of publication of application: **07.06.2023**
(21) Application number: 21212282.4
(22) Date of filing: 03.12.2021
(51) Int. Cl.: G16H 15/00, G16H 30/00, G16H 30/40, G16H 50/20

(54) **ENDOSCOPE IMAGE PROCESSING DEVICE**

(71) Applicant: Ambu A/S, 2750 Ballerup (DK)
(72) Inventor: ALONSO DÍAZ, Alejandro, 2750 Ballerup (DK); GADE, Josefine Dam, 1973 Frederiksberg (DK); JØRGENSEN, Andreas Härstedt, 2610 Rødovre (DK); LASSEN, Lee Herluf Lund, 2760 Måløv (DK); YU, Dana Marie, 2750 Ballerup (DK); NIELSEN, Morten Grønning, 2200 Copenhagen N (DK)
(74) Representative: AWA Denmark A/S

(57) **Abstract**

Disclosed is an image processing device for documenting a bronchoscopy procedure performed using an endoscope. The endoscope comprising an image captur-ing device, the image processing device comprising a processing unit operationally connectable to the image capturing device.The processing unit is configured to: obtain a model of the bronchial tree; obtain a stream of images captured by the image capturing device of the endoscope; continuously obtain estimates of the location of the endoscope in the model of the bronchial tree during the bronchoscopy; based on at least three estimates of the location of the endoscope in the model of the bronchial tree generate a report documenting the bronchoscopy procedure.

## Description

### Field

The present disclosure relates to an image processing device, a display unit, an endoscope system, a method for documenting a bronchoscopy procedure, and a computer program product.

### Background

Endoscopes are widely used in hospitals for visually examining body cavities and obtaining samples of tissue identified as potentially pathological. An endoscope typically comprises an image capturing device arranged at the distal end of the endoscope either looking forward or to the side. An endoscope is further typically provided with a working channel allowing a medical device such as a gripping device, a suction device, or a catheter to be introduced.

A bronchoscopy is an example of an important endoscopic procedure. During a bronchoscopy procedure an endoscope is advanced through the bronchial tree. This may however be a challenging task as parts of the bronchial tree is very narrow providing little space for the endoscope. Furthermore, navigating through the bronchial tree may be difficult.

To assist navigation, methods have been developed for determining the position of the endoscope in the bronchial tree.

The position of the endoscope in the bronchial tree may be found by firstly imaging the bronchial tree using CT or planar X-Ray, and then using a magnetic tracking system to determine the position of the endoscope in the CT image of a planar X-Ray image. The superDimension ^{™} Navigation System from Medtronic is an example of such a system.

Alternatively, the position of the endoscope may be estimated by processing the recorded endoscope images.

However, even with assist of systems for determining the position of the endoscope in the bronchial tree it may be difficult to perform a proper examination of the bronchial tree.

Furthermore, it may be difficult to document a procedure.

Thus, it remains a problem to provide an improved device / method for performing bronchoscopy procedures.

### Summary

According to a first aspect, the present disclosure relates to an image processing device for documenting a bronchoscopy procedure performed using an endoscope, the endoscope comprising an image capturing device, the image processing device comprising a processing unit operationally connectable to the image capturing device, wherein the processing unit is configured to:
obtain a model of the bronchial tree;
obtain a stream of images captured by the image capturing device of the endoscope;
continuously obtain estimates of the location of the endoscope in the model of the bronchial tree during the bronchoscopy;
based on at least three estimates of the location of the endoscope in the model of the bronchial tree generate a report documenting the bronchoscopy procedure.

Consequently, by obtaining a model of the bronchial tree and creating a report based on estimated location of the endoscope in the model, a bronchoscopy may effectively be documented.

The processing unit of the image processing device may be any processing unit, such as a central processing unit (CPU), a graphics processing unit (GPU), a microcontroller unit (MCU), a field-programmable gate array (FPGA), or any combination thereof. The processing unit may comprise one or more physical processors and/or may be combined by a plurality of individual processing units.

The image processing device may comprise a display, i.e. the image processing device may be integrated in a display unit. Alternatively / additionally, the image processing device may be operationally connectable to a separate display unit.

The model of the bronchial tree may be a generic model created to correspond to the anatomy of most bronchial tree. However, it may also be uniquely created for a particular patient e.g. based on other type of medical imaging such as CT, MRI or a previous bronchoscopy.

The location of the endoscope in the model of the bronchial tree may specify a particular part of the bronchial tree e.g. a particular branch of the bronchial tree. The location of the endoscope may be determined using any method such as magnetic tracking or image-based techniques such as machine learning methods e.g. as disclosed in PCT/EP2021/064985. The imaged based technique may attempt to recognize anatomical reference position in the endoscope images. Once an anatomical reference position has been recognized it may be determined that the endoscope is at the same anatomical reference position in the model of the bronchial tree.

The report may be generated after the endoscopic procedure has been concluded. Alternatively / additionally, the report may be generated / updated continuously during the bronchoscopy and displayed to the medical professional during the bronchoscopy e.g. in connection with endoscope images captured by the image capturing device. The report may be stored in a memory of the image processing device. The report may be stored in a single data file. Alternatively, different elements of the report may be stored in different data files. The report may comprise or consist of one or more images. The report may be automatically generated e.g. without user input. The report may be based on more than at least three estimates of the location of the endoscope in the model of the bronchial tree e.g. the report may be based on all of the obtained estimates of the location of the endoscope in the model of the bronchial tree. The report may comprise a quality measure of the bronchoscopy. The quality measure may be generated based on the at least three estimates of the location of the endoscope in the model of the bronchial tree. The quality measure may indicate a percentage of the bronchial tree that has been investigated. The percentage may be calculated relative to the full bronchial tree or a part of the bronchial tree dependent on the particular type of bronchoscopy being performed. The quality measure may further be based on the time used for performing the bronchoscopy.

In some embodiments, the report indicates the locations that have been visited by the endoscope during the bronchoscopy procedure.

In some embodiments, the report comprises an image of the model of the bronchial tree, where the parts of the model of the bronchial tree that have been visited by the endoscope are marked in the image of the model of the bronchial tree.

Consequently, an effective way of documenting a bronchoscopy is provided.

As an example the parts of the bronchial tree that have not been visited by the endoscope may be displayed with a first color and the parts of the bronchial tree that have been visited may be shown with a second color different from the first color. One or more of the parts that have been visited may be a part where no part of the endoscope is present e.g. a part from where the endoscope has been withdrawn.

In some embodiments, the processing unit is configured to store in response to a user input an image of the stream of images together with first data indicative of the location of the endoscope in the model of the bronchial tree when the image was recorded, and wherein the generated report comprises the image and the first data.

Consequently, it may be effectively documented where the endoscope was present when the image was captured. As an example, if the image shows a potential pathological structure, and a test on sample subsequently confirms that the structure is pathological, the report may be used to allow the medical professional to easily revisit the structure and thereby secure effective treatment.

The user input may be received from the handle of the endoscope e.g. the handle may comprise an input unit that can be used for capturing images. The input unit may be operationally connectable to the processing unit. Alternatively / additionally, the user input may be received from a display showing the endoscope images.

In some embodiments, the processing unit is configured to store a first stream of images, the first stream of images being at least a part of the stream of images and for a plurality of images of the first stream of images store second data indicative of the locations of the endoscope in the model of the bronchial tree when the plurality of images where recorded, and wherein the generated report comprises the first stream of images and the second data.

Today most medical professionals prefer to use endoscope still images when documenting procedures as it too complex and time consuming to navigate through a video consisting of a stream of images. However, by storing the second data logging the locations of the endoscope, it may become easier to navigate in a clinical video.

The first stream of images may represent the entire bronchoscopy. Alternatively, the first stream of images may represent only a part of the bronchoscopy. The first stream of images may be recorded in response to a user input from input unit e.g. in response to an input unit at the endoscope handle.

In some embodiments, the processing unit is operationally connectable to a display wherein the processing unit is configured to control the display to display the generated report.

In some embodiments, the processing unit is operationally connectable to an input unit, and wherein the processing unit is configured to:
control the display to display an image of the model of the bronchial tree;
receive an input signal from the input unit indicating part of the model of the bronchial tree;
based on the input signal and the second data identify a first image of the first stream of images that is recorded at the indicated part of the model of the bronchial tree or in proximity to the indicated part; and
control the display to display the first image.

Consequently, a medical professional may effectively navigate through a clinical video.

If the endoscope twice has visited the indicated part, then two images may be shown. As an example, if the indicated part is a part of the upper bronchial tree, the endoscope may have visited the part both when the endoscope enters the bronchial tree and is withdrawn from the bronchial tree.

In some embodiments, the processing unit is configured to control the display to start replaying the first stream of images from the first image.

The processing unit may be configured to replay the first stream of images directly or in a response to a further user input.

In some embodiments, the display is a touch display incorporating the input unit.

According to a second aspect, the present disclosure relates to a display unit for displaying images obtained by an image capturing device of an endoscope, wherein the display unit comprises an image processing device as disclosed in relation to the first asepct.

According to a third aspect, the present disclosure relates to an endoscope system comprising an endoscope and an image processing device as disclosed in relation to the first aspect of the present disclosure,
wherein the endoscope has an image capturing device and the processing unit of the image processing device is operationally connectable to the image capturing device of the endoscope.

In some embodiments, the image processing device forms part of a display unit as disclosed in relation to the second aspect of the disclosure.

According to a fourth aspect, the present disclosure relates to a method for documenting a bronchoscopy procedure performed using an endoscope, the endoscope comprising an image capturing device, wherein the method comprises:
obtaining a model of the bronchial tree;
obtaining a stream of images captured by the image capturing device of the endoscope;
continuously obtaining estimates of the location of the endoscope in the model of the bronchial tree during the bronchoscopy; and
based on at least three estimates of the location of the endoscope in the model of the bronchial tree generating a report documenting the bronchoscopy procedure.

According to a fifth aspect, the present disclosure relates to computer program product comprising program code means adapted to cause a data processing system to perform the steps of the method as disclosed in relation to the fourth aspect, when said program code means are executed on the data processing system.

In some embodiments, said computer program product comprises a non-transitory computer-readable medium having stored thereon the program code means.

The different aspects of the present disclosure can be implemented in different ways including image processing devices, display units, endoscope systems, method for documenting a bronchoscopy procedure, and computer program products described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1 shows an example of an endoscope.
Fig. 2 shows an example of a display unit that can be connected to the endoscope shown in Fig. 1.
Fig. 3 shows a schematic drawing of an image processing device according to an embodiment of the disclosure.
Fig. 4 shows a flowchart of a method for documenting a bronchoscopy procedure according to an embodiment of the disclosure.
Figs. 5 shows schematically a a report documenting the bronchoscopy procedure according to an embodiment of the disclosure.
Fig. 6a-b show images of a model of the bronchial tree according to an embodiment of disclosure.
Fig. 7ab shows endoscope images recorded during a bronchoscopy procedure according to an embodiment of the disclosure
Fig. 8 illustrates schematically how an embodiment of the disclosure may be used to effectively navigate through a clinical video

### Detailed description

In the following description, reference is made to the accompanying figures, which show by way of illustration how the embodiments of the present disclosure may be practiced.

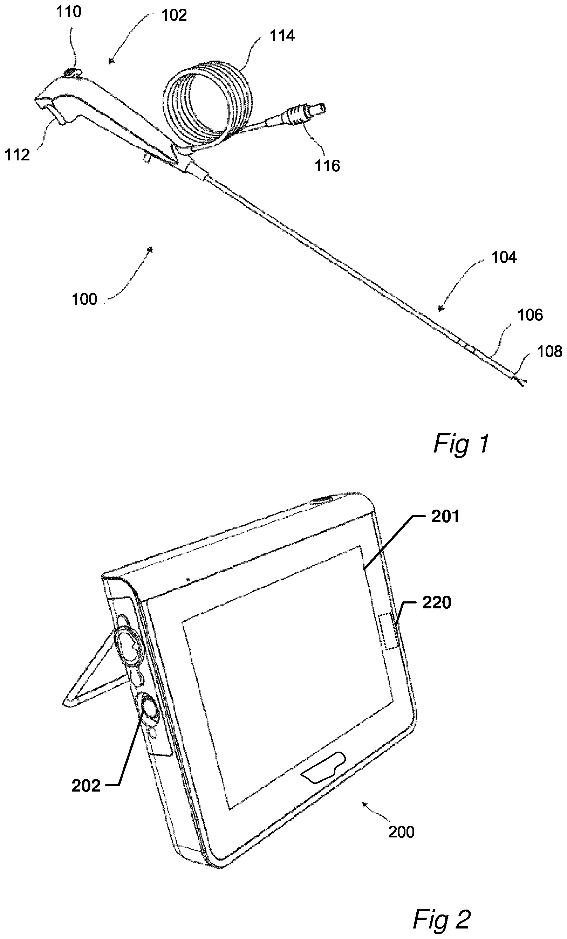

Fig. 1 illustrates an example of an endoscope 100. This endoscope may be adapted for single-use. Alternatively, the endoscope may be adapted for cleaning/sterilization and re-use. The endoscope 100 is provided with a handle 102 attached to an insertion tube 104 provided with a bending section 106. The endoscope 100 may also be designed without a bending section 106. The illustrated endoscope 100 is a flexible endoscope with a flexible insertion tube 104, but the principle of the disclosure can also be used with any type of endoscope. The insertion tube 104 as well as the bending section 106 may be provided with one or several working channels such that instruments, such as a gripping device or a catheter, may extend from the tip and be inserted into a human body via the endoscope. One or several exit holes of the one or several channels may be provided in a tip part 108 of the endoscope 100. In addition to the exit holes, a camera sensor, such as a CMOS sensor or any other image capturing device, as well as one or several light sources, such as light emitting diodes (LEDs), fiber, or any other light emitting devices, may be placed in the tip part 108. By having the camera sensor and the light sources and a display unit 200, illustrated in Fig. 2, configured to display images based on image data captured by the camera sensor, an operator is able to see and analyse an inside of the human body in order to for instance localize a position for taking a sample. In addition, the operator will be able to control the instrument in a precise manner due to the provided visual feedback. Further, since some diseases or health issues may result in a shift in natural colours or other visual symptoms, the operator is provided with valuable input for making a diagnosis based on the image data provided via the image capturing device and the display unit 200. The display unit comprises a display 201, a processing unit 220 (only schematically shown), and a connection port 202 for operationally connecting the endoscope to the processing unit 220. The connection port 202 may further be used to provide power to the endoscope.

In order to make it possible for the operator to direct the camera sensor such that different field of views can be achieved, the endoscope has a bending section 106 that can be bent in different directions with respect to the insertion tube 104. The bending section 106 may be controlled by the operator by using a knob 110 placed on the handle 102. The handle 102 illustrated in Fig. 1 is designed such that the knob 110 is controlled by a thumb of the operator, but other designs are also possible. In order to control a gripping device or other device provided via a working channel a push button 112 may be used. The handle 102 illustrated in Fig. 1 is designed such that a index finger of the operator is used for controlling the gripping device, but other designs are also possible.

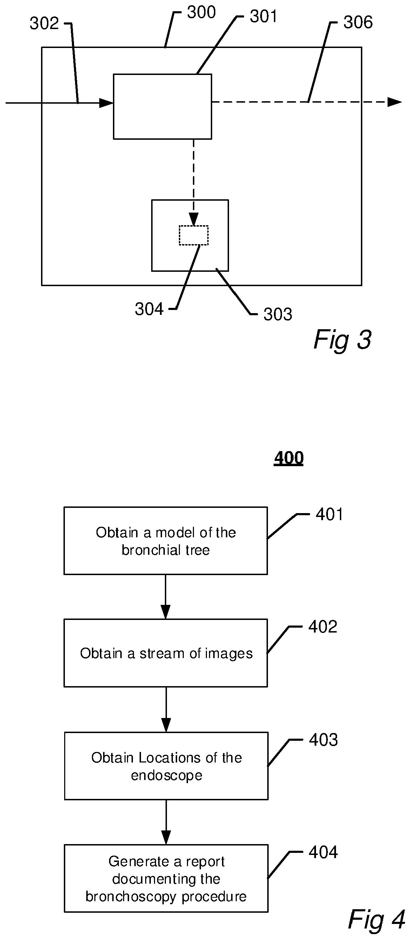

Fig. 3 shows a schematic drawing of an image processing device 300 for documenting a bronchoscopy procedure performed using an endoscope (not shown) according to an embodiment of the disclosure. The endoscope comprises an image capturing device. The image processing device 300 comprises a processing unit 301 operationally connectable 302 to the image capturing device. The processing unit is configured to obtain a model of the bronchial tree and a stream of images captured by the image capturing device of the endoscope. The processing unit may be wired connectable to the image capturing device. As an example, the image processing device may comprise a connection port for operationally connecting the endoscope to the processing unit 301. The connection port may further be used to provide power to the endoscope. Alternatively, the processing unit 301 may be wireless connectable to the image capturing device e.g. the image processing device 300 may comprise a wireless communication unit (not shown) configured to receive images from the image capturing device. The processing unit 301 is further configured to continuously obtain estimates of the location of the endoscope in the model of the bronchial tree during the bronchoscopy, and based on at least three estimates of the location of the endoscope in the model of the bronchial tree generate a report 304 documenting the bronchoscopy procedure. The report 304 may be stored in the memory unit 303. Alternatively / additionally, the report may be provided 306 to a display (not) shown to be displayed to an operator, possibly together with endoscope images.

Fig. 4 shows a flowchart of a method 400 for documenting a bronchoscopy procedure performed using an endoscope, the endoscope comprising an image capturing device. In 401, a model of the bronchial tree is obtained. In 402, a stream of images captured by the image capturing device of the endoscope is obtained. In 403, estimates of the location of the endoscope in the model of the bronchial tree during the bronchoscopy are continuously obtained. In step 404, based on at least three estimates of the location of the endoscope in the model of the bronchial a report documenting the bronchoscopy procedure is generated.

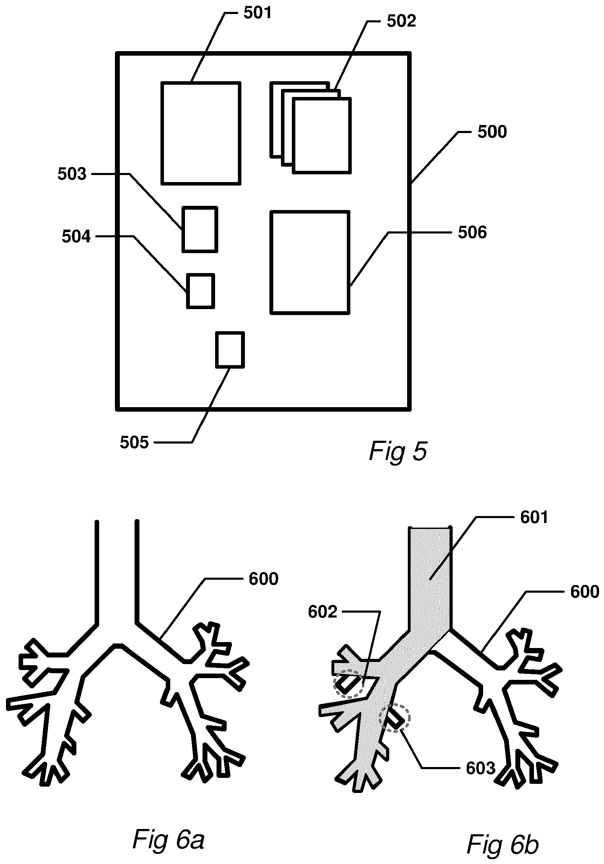

Fig. 5 shows schematically a report 500 documenting the bronchoscopy procedure according to an embodiment of the disclosure. The report comprises an image of the model of the bronchial tree 501, where the parts of the model of the bronchial tree that have been visited by the endoscope is marked in the image of the model of the bronchial tree. Thus, the image 501 indicates the locations that have been visited by the endoscope during the bronchoscopy procedure. The report 500 may further comprise one or more images 502 of a stream of images. For each image of the one or more images 502 the report 500 further comprises first data 503 indicative of the location of the endoscope in the model of the bronchial tree when that image was recorded. The report 500 may further comprise a quality measure 504 of the bronchoscopy. The report may further comprise a first stream of images 506 and second data 505 indicative for a plurality of images of the first stream of images 506 the locations of the endoscope in the model of the bronchial tree when the plurality of images where recorded.

Fig. 6a-b show images 601 of a model of the bronchial tree according to an embodiment of the disclosure. In other embodiments, the images of the model of the bronchial tree may be provided with more or fever details. The model of the bronchial tree may be a generic model created to correspond to the anatomy of most patients. However, the model may also be uniquely created for a particular patient e.g. based on other type of medical imaging such as CT, MRI or a previous bronchoscopy. Fig. 6a shows the image before a bronchoscopy has been started, where all parts of the bronchial tree are shown in a first colour (white). Fig. 6b shows the image after a bronchoscopy of the right part of the bronchial tree has been performed (the right part of the bronchial tree is normally shown to the left in an anatomical representation of the bronchial tree). In. Fig. 6b the parts 601 of the model of the bronchial tree that have been visited by the endoscope is marked in the image of the model of the bronchial tree with a second color (gray). It can be seen that two parts of the bronchial tree have been missed 602 603. Thus, if the medical professional is presented with a report comprising the image in Fig. 6b, the medical professional may recognize that the two parts 602 603 must be examined to prevent that any pathological conditions are overlooked.

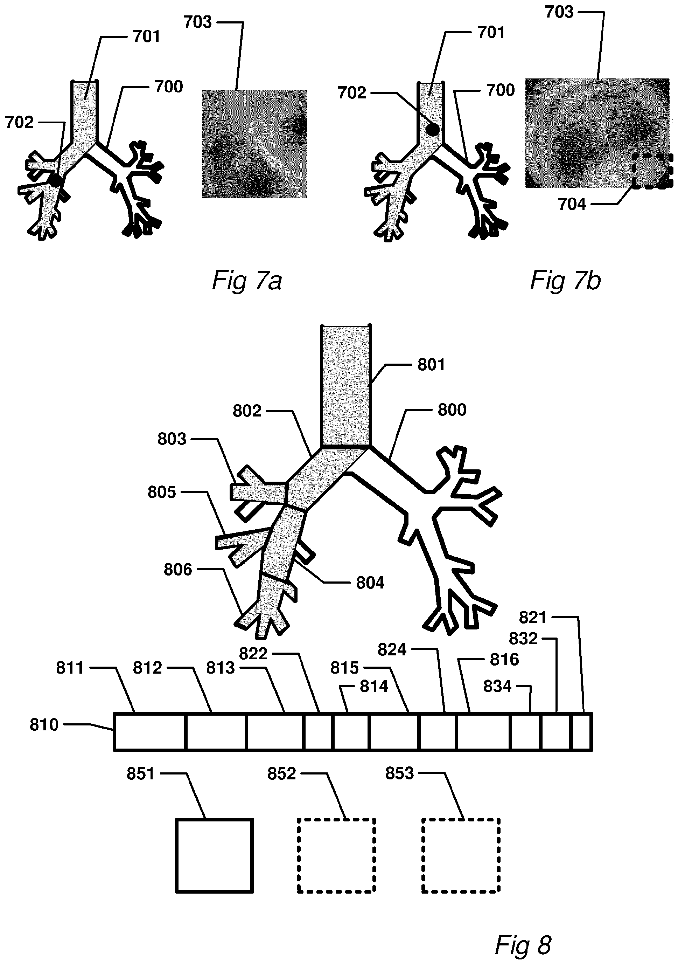

Fig. 7a shows an endoscope image 703 recorded during a bronchoscopy procedure according to an embodiment of the disclosure. Shown is also an image of a model of the bronchial tree 700, where the parts of the model of the bronchial tree that have been visited by the endoscope is marked 701 in the image of the model of the bronchial tree. In the image of the model of the bronchial tree 700 is shown a dot 702 illustrating where the endoscope was located when the image 703 was recorded. The position of the dot may be determined by first data forming part of a report documenting the bronchoscopy procedure.

Fig. 7b corresponds to Fig. 7a with the difference that the image 703 is recorded at another position 702. Furthermore, the box 704 schematically illustrates that the endoscope image 703 may be augmented with the image of the model of the bronchial tree 700. As an example, the image of the model of the bronchial tree 700 may be overlayed the endoscope image 703 in the corner 704. The image of the model of the bronchial tree 700 may be made semi-transparent before being overlayed the endoscope image 703.

Fig. 8 illustrates schematically how an embodiment of the disclosure may be used to effectively navigate through a clinical video. Shown is a model of the bronchial tree 800. The model has been divided into a number of parts 801-806. In this embodiment, only the right part of the bronchial tree is being investigated. Thus, only the right part of the bronchial tree has been divided into parts. However, in other embodiments, the entire bronchial tree is being investigated, whereby also the right part will be divided into parts. There is also shown a first stream of images 810 representing the entire bronchoscopy. During the bronchoscopy for a plurality of images of the first stream of images 810 second data is stored indicative of the locations of the endoscope in the model of the bronchial tree when the plurality of images where recorded. As an example, the second data may be generated for the first image recorded after the endoscope has entered a new part of the bronchial tree. Thus, the second data may be stored for the first image recorded after the endoscope has entered the parts 801-806. The second data may be used to divide the first stream of images 810 into a plurality of sections 811-816, 821, 822, 824, 834, 832. In the section 811 the endoscope is examining / propagating through the part 801 of the bronchial tree, in the section 812 the endoscope is examining / propagating through the part 802 of the bronchial tree, in the section 813 the endoscope is examining / propagating through the part 803 of the bronchial tree, in the section 822 the endoscope is revisiting the part 802 of the bronchial tree, in the section 814 the endoscope is examining / propagating through the part 804 of the bronchial tree, in the section 815 the endoscope is examining / propagating through the part 805 of the bronchial tree, in the section 824 the endoscope is revisiting the part 804 of the bronchial tree, in the section 816 the endoscope is examining / propagating through the part 806 of the bronchial tree, in the section 834 the endoscope is revisiting the part 804 of the bronchial tree, in the section 832 the endoscope is revisiting the part 802 of the bronchial tree, and in the section 821 the endoscope is revisiting the part 801 of the bronchial tree.

In some embodiments, the model of the bronchial tree 800 may be displayed and an input signal may be received indicating a part of the model of the bronchial tree. Based on the input signal and the second data a first image of first stream of images 810 that is recorded at the indicated part of the model of the bronchial tree or in proximity to the indicated part may be found and displayed. As an example, the model of the bronchial tree 800 may be displayed on a touch display, where a user is provided with the possibility to select any one of parts 801-806. If the user select part 801, the first image from section 811 may be displayed in a first part 851 of the touch display and the first image from the section 821 may be displayed in a second part 852 of the touch display. The user may then select one of the images whereby either the section 811 or the section 821 of the first stream is being replayed. Correspondingly, if the user selects part 802, the first image from section 812 may be displayed in the first part 851 of the touch display, the first image from the section 822 may be displayed in the second part 852 of the touch display, and the first image from the section 832 may be displayed in a third parts 853 of the display. The user may then select one of the images whereby either the section 812, the section 822 or the section 832 of the first stream is being replayed.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. An image processing device for documenting a bronchoscopy procedure performed using an endoscope, the endoscope comprising an image capturing device, the image processing device comprising a processing unit operationally connectable to the image capturing device, wherein the processing unit is configured to:
obtain a model of the bronchial tree;
obtain a stream of images captured by the image capturing device of the endoscope;
continuously obtain estimates of the location of the endoscope in the model of the bronchial tree during the bronchoscopy;
based on at least three estimates of the location of the endoscope in the model of the bronchial tree generate a report documenting the bronchoscopy procedure.

2. An image processing device according to claim 1, wherein the report indicates the locations that have been visited by the endoscope during the bronchoscopy procedure.

3. An image processing device according to claims 1 or 2, wherein the report comprises an image of the model of the bronchial tree, where the parts of the model of the bronchial tree that have been visited by the endoscope are marked in the image of the model of the bronchial tree.

4. An image processing device according to any one of claims 1 to 3, wherein the processing unit is configured to store in response to a user input an image of the stream of images together with first data indicative of the location of the endoscope in the model of the bronchial tree when the image was recorded, and wherein the generated report comprises the image and the first data.

5. An image processing device according to any one of claims 1 to 4, wherein the processing unit is configured to store a first stream of images, the first stream of images being at least a part of the stream of images and for a plurality of images of the first stream of images store second data indicative of the locations of the endoscope in the model of the bronchial tree when the plurality of images where recorded, and wherein the generated report comprises the first stream of images and the second data.

6. An image processing device according to any one of claims 1 to 5, wherein the processing unit is operationally connectable to a display wherein the processing unit is configured to control the display to display the generated report.

7. An image processing device according to claims 5 and 6, wherein the processing unit is operationally connectable to an input unit, and wherein the processing unit is configured to:
control the display to display an image of the model of the bronchial tree;
receive an input signal from the input unit indicating part of the model of the bronchial tree;
based on the input signal and the second data identify a first image of first stream of images that is recorded at the indicated part of the model of the bronchial tree or in proximity to the indicated part; and
control the display to display the first image.

8. An image processing device according to claims 7, wherein the processing unit is configured to control the display to start replaying the first stream of images from the first image.

9. An image processing device according to claims 8 or 7, wherein the display is a touch display incorporating the input unit.

10. A display unit for displaying images obtained by an image capturing device of an endoscope, wherein the display unit comprises an image processing device according to any one of claims 1 to 9.

11. An endoscope system comprising an endoscope and an image processing device according to any one of claims 1 to 9, wherein the endoscope has an image capturing device and the processing unit of the image processing device is operationally connectable to the image capturing device of the endoscope.

12. An endoscope system according to claim 11, wherein the image processing device forms part of a display unit according to claim 10.

13. A method for documenting a bronchoscopy procedure performed using an endoscope, the endoscope comprising an image capturing device, wherein the method comprises:
obtaining a model of the bronchial tree;
obtaining a stream of images captured by the image capturing device of the endoscope;
continuously obtaining estimates of the location of the endoscope in the model of the bronchial tree during the bronchoscopy; and
based on at least three estimates of the location of the endoscope in the model of the bronchial tree generating a report documenting the bronchoscopy procedure.

14. A computer program product comprising program code means adapted to cause a data processing system to perform the steps of the method according to claim 13, when said program code means are executed on the data processing system.

15. A computer program product according to claim 14, wherein said computer program product comprises a non-transitory computer-readable medium having stored thereon the program code means.
